# EUROPEAN PATENT APPLICATION

(11) **EP 4 098 287 A1**
(43) Date of publication of application: **07.12.2022**
(21) Application number: 21177646.3
(22) Date of filing: 03.06.2021
(51) Int. Cl.: A61L 27/26, A61L 27/50, B33Y 70/00

(54) **COMPOSITION AND SCAFFOLD MATERIAL TO REGENERATE CARTILAGES**

(71) Applicant: Allegro NV, 9050 Gent (BE)
(72) Inventor: Schelfhout, Jorg, 9050 Gent (BE); Decuypere, Lucas, 9050 Gent (BE)
(74) Representative: Roux, Stéphane

(57) **Abstract**

A composition to produce scaffold material adapted to regenerate cartilage, the material including:
• Functionalized nanocrystalline cellulose including a plurality of first reactive moieties,
• Functionalized gelatine peptide chains including a plurality of second reactive moieties adapted to react with the first reactive moieties.

## Description

The present description relates to the field of tissue engineering and discloses compositions, materials and methods that may be adapted to regenerate cartilages.

Due to the prevalence of cartilage and meniscus injuries, the importance of tissue engineering (TE) in their treatment significantly increases. Since the self-healing capabilities of the cartilage are fairly limited, injuries often lead to degenerative changes. In many cases, total or partial meniscectomy is the only available treatment option, which has poor long-term prognosis with a high risk of cartilage degeneration. Therefore, the need for innovative treatment methods is urgent.

3D printing is an advantageous technique for this objective as it allows replication of the overall meniscus shape for generation of patient-specific scaffolds and fine control of the internal fibre architecture.

However, current scaffolds obtained by 3D printing usually produce low quality regenerated tissues or have poorly defined dimensions. There is thus a need to improve the current situation.

This present disclosure relates to a composition to produce scaffold material adapted to regenerate cartilages, the composition including:
- Functionalized nanocrystalline cellulose including a plurality of first reactive moieties,
- Functionalized gelatine peptide chains including a plurality of second reactive moieties adapted to react with the first reactive moieties.

Such a composition shows superior performance as a bio-ink for 3D printing. In particular, it allows to obtain rheological properties (i.e., flow behaviour) showing shear thinning and thixotropic behaviours, which allows to obtain printed shapes with a high dimensional control. Further, such a printed shape can be easily cross-linked into a scaffold having a high porosity and high degree of pore interconnectivity, which may provide a high level of cartilages regeneration.

Advantageously, the first reactive moiety is a nucleophile, for example chosen among an amine, a stabilized carbanion, a thiolactone and preferably a thiol. Such a nucleophile may be stable before cross-linking and may lead to an efficient cross-linking reaction.

Advantageously, the functionalized nanocrystalline cellulose comprises a plurality of linkers bearing the first reactive moieties and chosen among an amino acid, a peptide, cysteamine, 3-amino-1,2,4-triazole-5-thiol or 6-amino-1-hexanethiol; cysteamine being preferred. Such linkers are easily available and provide an efficient link to the first reactive moiety. They can be easily coupled to the nanocrystalline cellulose by an extremity of the cellulose chains, may contribute to the gelation properties of the composition and show good biocompatibility.

Advantageously, the linker is an amino acid and preferably a cysteine, for example a L-cysteine. Such a linker shows good biocompatibility and contributes to the gelation properties of the composition.

Advantageously, the functionalized nanocrystalline cellulose is under the form of crystals including faces and the first reactive moieties are located on a single face of each of the crystals. Such crystals provide superior rheological properties and may contribute to the porosity of the scaffold after cross-linking.

Advantageously, each of the second reactive moieties of the functionalized gelatine peptide chains comprises a double bond or a triple bond, for example a carbon-carbon double bond or carbon-carbon triple bond, preferably activated. Such second reactive moieties allow an efficient click or photo-click reaction with the first reactive moieties.

Advantageously, the second reactive moieties of the functionalized gelatine peptide chains are chosen among norbornene, norbornene-derivative, acrylate, methacrylate, α,β-unsaturated aldehyde or ketone, vinyl ester, vinyl sulfone, imidazole and maleimide.

Advantageously, the weight percentage of functionalized gelatine peptide chains with regard to the composition is at most 10 %, preferably at most 5 %.

Advantageously, the weight percentage of functionalized cellulose with regard to the composition 0.1 to 15 %, preferably 0.5 to 10 % and again preferably 1 to 5 %.

Another aspect of the present disclosure is a scaffold material adapted to regenerate cartilages, the material including:
- Functionalized gelatine peptide chains
- Functionalized nanocrystalline cellulose
- A plurality of bridges cross-linking the functionalized nanocrystalline cellulose and the functionalized gelatine peptide chain.

Advantageously, each bridge comprises at least one sulphur atom and/or at least one amino acid, for example at least one cysteine.

Such a scaffold for cartilage may allow a high porosity with an interconnected pore network which ensures optimal cell growth and transportation of nutrients. In addition, such a high porosity may be obtained using a low concentration of hydrogel composition while maintaining good dimensions of a printed shape.

For example, the bridges result from the coupling of the first reactive moieties, possibly connected to the cellulose chains by a linker, with the second reactive moieties, as above described. Preferably, the above scaffold may be obtained from the above composition.

Advantageously, the functionalized nanocrystalline cellulose is under the form of crystals including faces and wherein the bridges are connected to a single face of each of the crystals.

Another aspect of the present disclosure is a method to form a scaffold adapted to regenerate cartilage, the method including:
- Providing a composition as described above,
- Forming a scaffold shape
- Cross-linking the formed scaffold shape into a scaffold.

Advantageously, the step of forming a scaffold shape is performed by 3D-printing and the step of cross-linking is performed by a click or photo-click reaction, which allow a fast and efficient production of the scaffold.

Another aspect of the present disclosure is a method to form a scaffold adapted to regenerate cartilage, the method including:
- Providing a composition as described above,
- Cross-linking the composition into a scaffold material.
- Forming a scaffold from the scaffold material.

For example, the step of forming could be performed by cutting, moulding or extruding the scaffold material.

Another aspect of the present disclosure is a method to regenerate cartilage, including forming a scaffold as described above and implanting such a scaffold into a body, such as a mammal body, for example in a wounded joint. The scaffold may then provide an efficient structural support for cell proliferation, maintenance of cell function, and extracellular (ECM) formation. For example, the step of implanting is performed through arthroscopy.

Further advantages and preferred embodiments of the present disclosure will become apparent from the following detailed description and drawings, in which:
Figure 1 shows the preparation of crystalline nanocellulose from natural cellulose.
Figure 2 is a schematic representation of cellulose chains within nanocrystalline cellulose.
Figure 3 shows a typical structure of gelatine.
Figure 4 shows a scaffold material according to the present disclosure.

The present disclosure includes a composition for tissue engineering, in particular a composition that may be adapted for 3D printing a scaffold or for producing a scaffold material.

The composition may comprise at least two components: cellulose and gelatine. However, other components may be added such as purified water, growth factors, proteins, hyaluronic acid and/or polycaprolactone.

Cellulose may be nanocrystalline cellulose, under the form of cellulose crystals. The cellulose crystals may have a bar shape comprising distinct faces, such as six faces and dimensions of 100 to 900 nm in length, preferably 150 to 850 nm, again preferably 240 to 760 nm, 370 to 620 nm, 410 to 580 nm and at most preferably around 500 nm. The width and height of the crystals may be 5 to 40 nm, preferably 9 to 32 nm, again preferably 10 to 25 nm, and most preferably 15 to 20 nm. Such dimensions may be obtained from electron microscopy, such as scanning electron microscopy (SEM) or preferably transmission electron microscopy (TEM).

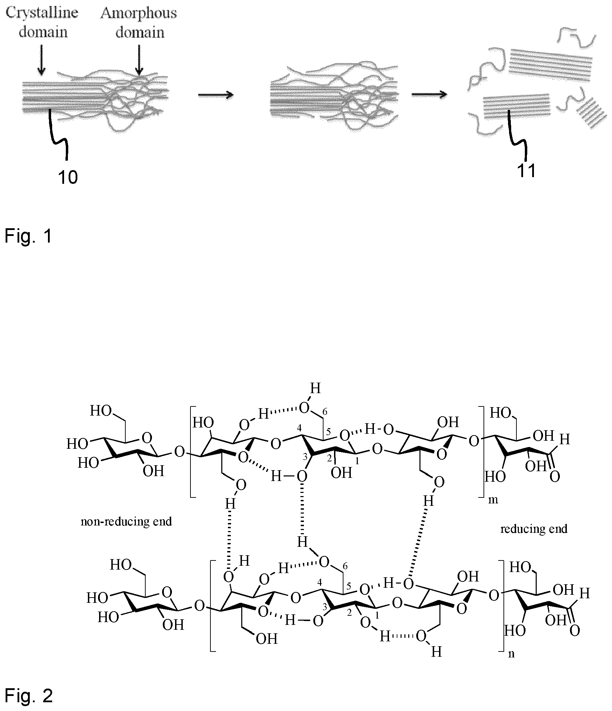

Nanocrystalline cellulose may be obtained by hydrolysis according to Reaction 1 below and Fig. 1.

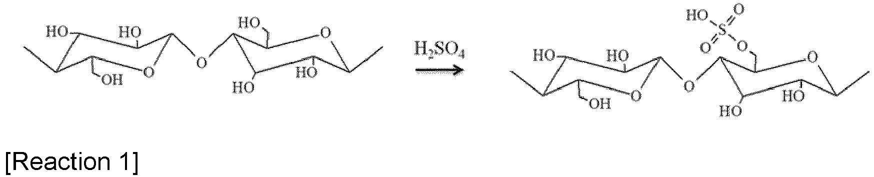

In Fig. 1, cellulose 10, for example extracted from cotton or from a bacteria source, may comprise crystalline and amorphous domains and the amorphous domains may be cut by acidic hydrolysis, for example using sulfuric acid in order to obtain only the crystalline domains, preferably under the form of nanocrystals 11. Depending on the reaction conditions, different shapes of nanocrystals may be obtained while the bar shape may provide favourable rheological properties to the composition.

The cellulose may be functionalized by the addition of first reactive moieties. Consequently, the first reactive moieties may differ from the reactive moieties of natural and/or nanocrystalline cellulose. Preferably, the first reactive moieties are inserted at an extremity of the cellulose chains, for example on the aldehyde extremities of the cellulose chains (so-called reducing end). Since the cellulose chains may be aligned within a nanocrystal, these extremities may correspond to a single face of each nanocrystal, for example an extremal face of the bar-shaped nanocrystals. The cellulose chains may be maintained within the nanocrystals by intra and inter-molecular hydrogen bonds, for example as represented in Fig. 2.

The first reactive moieties may be a nucleophile, for example chosen among an amine, a stabilized carbanion, a thiolactone and preferably a thiol. Preferably, the first reactive moieties are inserted at the aldehyde extremities of the cellulose chains by reaction of the aldehyde moieties with one or several linkers, for example including an amino acid or a peptide. The amino acid or peptide may be natural or artificial. Preferably, the first reactive moiety is a thiol -SH group and is inserted by coupling cysteine or cysteamine to the aldehyde extremities by a coupling reaction, for example a reductive amination, according to Reactions 2.

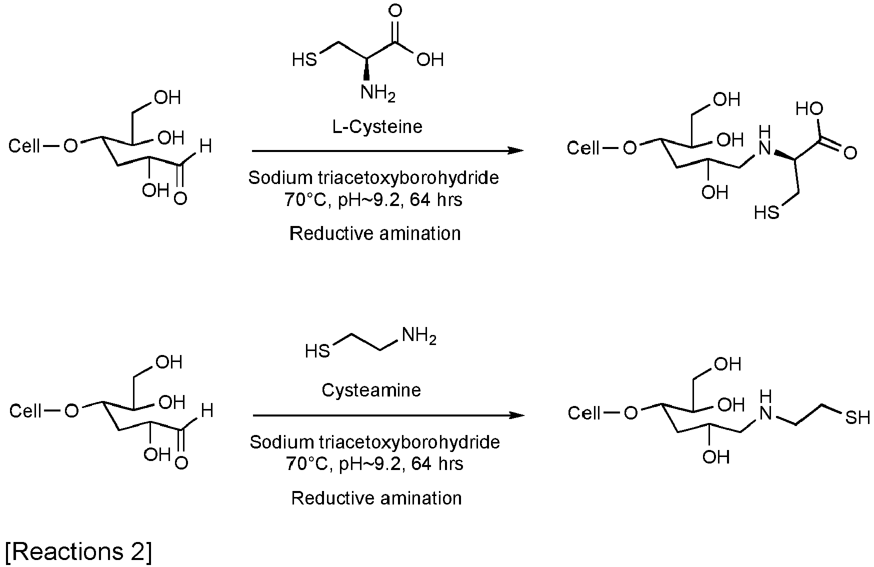

According to Reactions 2, a reductive amination may be performed by Triacetate Borohydride with an alkaline pH, for example greater than 8 or greater than 9 and at a temperature higher than room temperature, for example 50 °C or more, 60 or 70 °C. The alkaline buffer may be sodium carbonate. The reaction time may be long and may be greater than 24 h, for example greater than 38 h, preferably around 48 h or 50 h and up to 64 h. Purification may be performed through centrifugation and washings cycles or other methods known in the art. Such reaction conditions may allow to obtain functionalized nanocrystalline cellulose without destroying the nanocrystals. Preferably, the degree of functionalization, i.e. the thiol content of the functionalized nanocrystalline cellulose, as determined by Ellam assay is 1 to 10 µmol/g.

Other linkers may also be used, as a molecule or in the form of a salt such as a biocompatible salt, for example a hydrochloride salt. For example, the amino acid may be chosen in the list comprising cysteine, homocysteine and homocysteine thiolactone. Non-amino acid linkers may include preferably cysteamine but also 3-amino-1,2,4-triazole-5-thiol or 6-amino-1-hexanethiol. Preferably, the linker comprises at least an amine moiety and a nucleophilic moiety as a first reactive moiety, separated by at least one carbon atom, preferably at least 2 carbon atoms and preferably up to 10 carbon atoms, up to 8 carbon atoms, up to 6 carbon atoms and most preferably up to 4 carbon atoms. The nucleophilic moiety is preferably a thiol moiety.

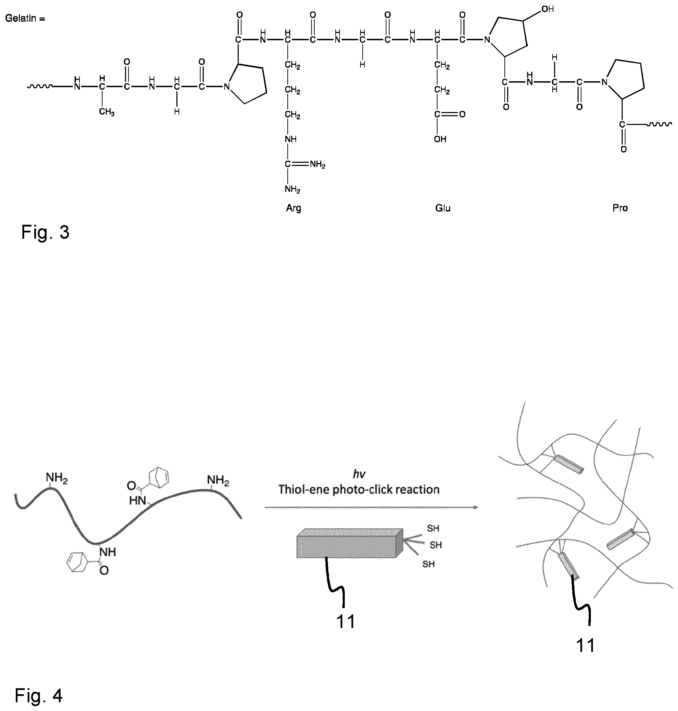

Gelatine may include gelatine peptide chains, preferably obtained from bovine gelatine because the iso-electric point of bovine gelatine is close from the iso-electric point of the human body. However, other type of gelatine may be used, in particular for non-human applications. Fig. 3 shows a structure of gelatine, for example showing Arginine, Glycine and Proline. However, other natural amino acids may be present in gelatine, as known by the skilled person.

Gelatine may be functionalized to insert second reactive moieties. Consequently, the second reactive moieties may differ from the reactive moieties of gelatine. For example, the second reactive moiety is or include a double bond or a triple bond, such as a carbon-carbon unsaturated bond, preferably activated, for example by hyperconjugation or conjugated with another unsaturated bond. Preferably, the carbon-carbon unsaturated bond may be constrained, for example thanks to a cycle. For example, the second reactive moiety is preferably norbornene or a norbornene derivative but may also include acrylate, methacrylate, α,β-unsaturated aldehyde or ketone, vinyl ester, vinyl sulfone, imidazole, and maleimide.

Norbornene may be added to gelatine using 5-norbornene-2-carboxylic acid, according to Reaction 3 below. In a first step, 5-norbornene-2-carboxylic acid may be reacted with EDC (1-Ethyl-3-(3-dimethylaminopropyl)carbodiimide) and NHS (N-Hydroxysuccinimide) in DMSO (Dimethyl sulfoxide). This reaction may be conducted at room temperature for a couple of hours or more and/or 8h or less, for example 5 h.

In a second step, the product of the first step may be coupled to Gelatine, for example in DMSO at a temperature of 40°C or more such as 50° C or more and for a reaction time of 8h or more such as 12h or 15h.

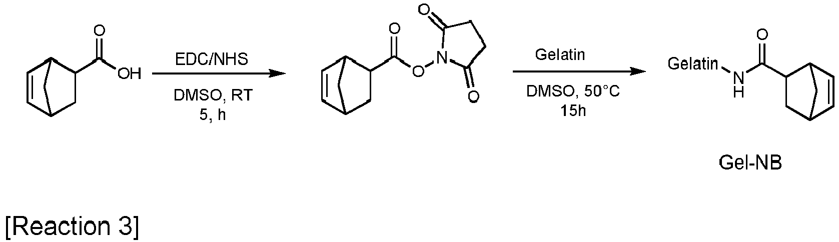

At the end of the Reaction 3, norbornene functionalized gelatine may be obtained. Norbornene is well adapted to react with the thiol groups of the nanocrystalline cellulose. Here, norbornene and thiol are only examples of first and second reactive moieties and other types of reactive moieties may be used provided they react with each other, preferably by a click or photo-click reaction. Preferably, the second reactive moiety may be of the type reacting with a thiol moiety, as a preferable first reactive moiety. A purification step may be performed after the above functionalizing Reaction 3 by any known methods, for example by dialysis.

The composition may be used as a ink or starting material to obtain 3D printed scaffolds. For example, the composition is a structural and functional ink. A shape corresponding to a broken part of a cartilage of a body may be formed, for example 3D printed using the above composition and cross-linked, in order to form a scaffold having a geometry adapted to support cells and/or cartilages, as known in the art. Thanks to the specific composition and in particular the nanocrystalline cellulose, the composition may have optimal properties for 3D printing or other forming method, for example optimal rheological properties. Preferably, the composition may have a thixotropic property and a fast viscosity recovery ability.

For example, the composition may comprise functionalized nanocrystalline cellulose in a range of 1 to 15 % w/w with regard to the total weight of the composition, preferably, 2 to 13 %, again preferably 5 to 11 %, 7 to 10 % or around 8 %. Further, the composition may comprise functionalized gelatine peptide chains in a range of 1 to 20 % w/w with regard to the total weight of the composition, preferably 3 to 18 %, again preferably 5 to 13 %, 7 to 12 % and around 10 %.

The rheological characterization of the composition was investigated using a Anton Paar rheometer MCR 302e in combination with an UV lightsource Omnicure S1500. Dynamic strain scan test: at a fixed frequency, we applied a strain scan from 0,1 to 100% to determine the linear viscoelastic range. Dynamic frequency scanning test: constant strain at 1% and a frequency scan from 0,1 to 100 rad.s⁻¹. Steady-state shear rate tests were performed at 5 - 25°C, and the shear rate ranged from 0,01 to 100 s⁻¹, and preferably was equal or around 20 s⁻¹.

The viscosities of the samples were measured by continuous measurement at different time points and viscosity recovery test. The viscosity of the composition may be between 10 and 300.000 mPa.s⁻¹, preferably 1700 to 240.000 mPa⁻¹, again preferably 11.000 to 160.000 mPa⁻¹, 48.000 to 115.000 mPa⁻¹, 82.000 to 104.000 mPa⁻¹.

The composition may also comprise distilled or purified water, as well as usual additives such as growth factors and proteins that may contribute to cartilage regeneration, for example human growth factors, Human connective tissue growth factor (CTGF), Transforming growth factor-β3, Platelet-rich fibrin (PRF) and/or polycaprolactone. In particular, PRF may enhance cellular migration and promote both meniscocyte proliferation and meniscocyte ECM secretion. Preferably, the additives are selected not to alter the rheological properties of the ink and/or the properties of the scaffold material.

The printing step involving the above composition as a ink may be performed with any adapted 3D-printer, in particular comprising one or two nozzles. The printing pressure may be 400 to 1000 kPa, preferably, 520 to 870 kPa, again preferably 660 to 740 kPa. The printing speed may be 0,2 to 150 mm/sec, preferably 8 to 130 mm/sec, again preferably 21 to 104 mm/sec, 43 to 87 mm/sec and for example around 72 mm/sec. Any adapted shape may be printed, preferably a shape adapted, close or identical to the shape of a part of the cartilage to be regenerated. The shape may also complement the part of the cartilage to be regenerated.

For example, a 3D printer with a dual nozzle and supporting multitemperature may be used to co-extrude the composition with polycaprolactone in order to enhance the mechanical properties of the printed shape. Alternatively or in combination, any other forming method known in the art may be used.

Cross-linking of the printed shape may be performed, preferably using a click reaction, in order to obtain a scaffold or at least scaffold material. For example the click reaction may be a click or a photo-click reaction triggered by UV-light. Any kind of adapted UV cross-linking method may be used, for example a mere exposure to a UV lamp emitting UVA, for example with a wavelength of 365 nm. Light- or laser-based techniques may include stereolithography (SLA), digital light projection (DLP) and two-photon polymerization (2PP)

For example, a photo initiator may be used, such as a type I photoinitiator such as LAP (Lithium(2,4,6-trimethylbenzoyl)phenylphosphinate - CAS: 85073-19-4). LAP may be cleaved according to Reaction 4 below, thus providing a reactive radical promoting the cross-linking reaction between the first reactive moieties and the second reactive moieties. Another photo initiator may be Irgacure 2959 (2-Hydroxy-4'-(2-hydroxyethoxy)-2-methylpropiophenone - CAS: 106797-53-9).

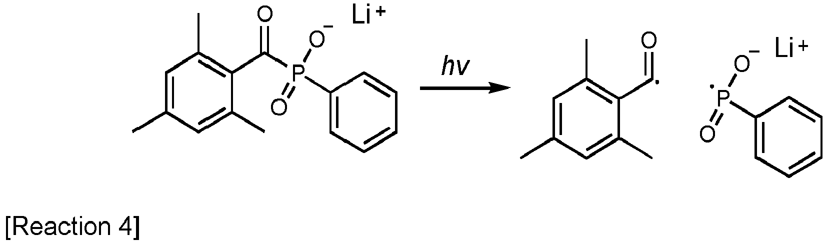

An example of UV cross-linking reaction is shown in Reaction 5 below, wherein R1 is cellulose or functionalized cellulose and R2 is gelatine or functionalized gelatine.

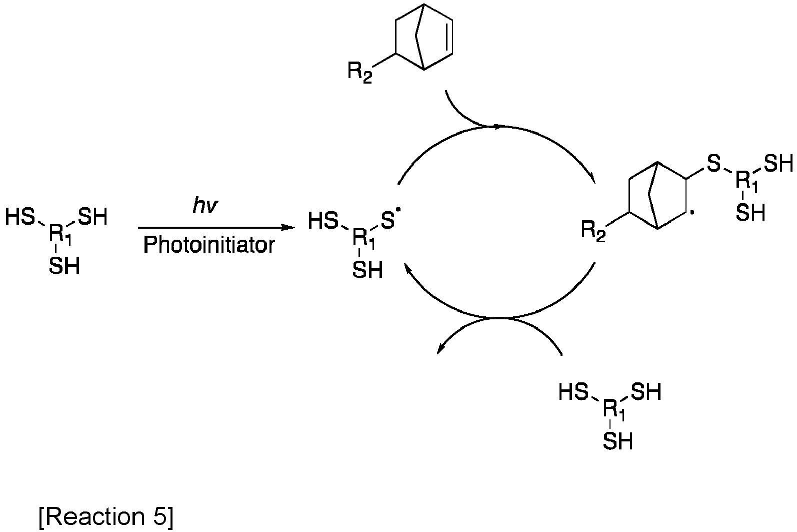

For example, the cross-linking step may be performed using 300-600 nm UV light and preferably 365 nm in the case of LAP, with a power of 2 mW to 6 W and during a time of 10 s to 30 min, preferably 45 s to 23 min, again preferably 76 s to 18 min, 3 min to 11 min and for example 7 min to 9 min. For example, the cross-linking step results in a gel fraction that may be at least 76 % w/w, preferably at least 82 %, again preferably at least 93 %.

Thanks to cross-linking, bridges are formed between nanocrystalline cellulose and gelatine, for example as represented in Fig. 4. This may form a 3-dimensional network that may enclose a liquid such as water. The bridges may include the first and second reactive moieties and the linker. The linker may have 3 to 20 atoms, preferably 5 to 15 atoms, 6 to 12 atoms, 7 to 10 atoms, for example 8 or 9 atoms. The linker may comprise a thiol atom and/or a cyclic or a bicyclic portion, for example of 4 to 12 carbon atoms, preferably saturated.

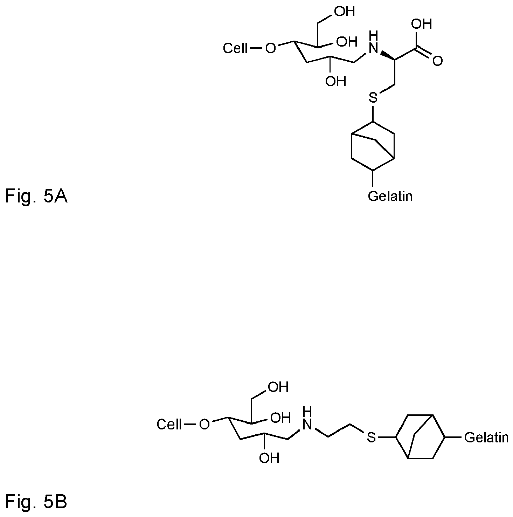

Examples of preferable bridges are provided in Figs. 5A-5B and may include the reducing end of cellulose ("Cell" in Figs. 5), cysteine (Fig. 5A) or cysteamine (Fig. 5B) and norbornane.

Alternatively, the composition may be cross-linked into scaffold material before the step of forming a scaffold. The scaffold may then be formed by any known method such as moulding, cutting or extrusion.

After cross-linking, a scaffold or a scaffold material is obtained, preferably under the form of a semi-solid material. This material may form a porous scaffold promoting cellular growth, in order to regenerate tissue or cartilages.

A universal tensile testing machine was used to measure the hydrogel mechanical strength via a uniaxial compression test method. The elastic modulus hydrogel samples were obtained by the ratio of stress and strain with a linear deformation range. The hydrogel samples were cylindrical and had a diameter of 15 ± 1 mm. The tests were performed at a compression speed of 0,5 mm s⁻¹.

The ultimate tensile strength UTS may be 40 to 500 kPa, preferably 72 to 460 kPa, again preferably 105 to 380 kPa and for example 225 to 318 kPa. The compressive stress of the scaffold material may be of 10 to 4100 kPa, preferably 81 to 3600 kPa, again preferably 230 to 2900 kPa, 540 to 2100 kPa, 680 to 1600 kPa, 980 to 1350 kPa. The young elastic modulus could be 100 to 4000 kPa, preferably 185 to 3450 kPa, again preferably 260 to 2900 kPa, 580 to 2200 kPa and for example 890 to 1750 kPa.

The microstructures of the porous scaffolds were observed using a scanning electron microscope. The cross-sections of the porous scaffolds were coated by a sputter coater before observation. The mean pore size and pore density of the porous scaffolds were measured from their scanning electron microscopy (SEM) images. The porosity of scaffolds was measured according to Archimedes' principle. The scaffold material may have preferably a porosity of 40 to 65 %, preferably 45 to 55 % and a high pore interconnectivity, preferably higher than 80 %, again preferably higher to 92 % or higher to 97 %, preferably 99 or 100 %.

The mechanical properties of the porous scaffolds were measured by a static compression mechanical test machine. Before testing, the samples were punched into cylindrical samples (8 mm diameter x 4 mm height) with a biopsy punch. Each test sample was compressed at a rate of 2.0 mm min⁻¹ at room temperature. The compressive modulus was calculated from the curves and the sample dimensions. The compression modulus may be 100 to 60.000 kPa, preferably 320 to 52.000 kPa, again preferably 640 to 32.500 kPa, 1260 to 26.000 kPa, 5680 to 21.600 kPa, 8900 to 17.000 kPa, 10.100 to 14.500 kPa.

After forming the scaffold, the scaffold may be implanted in the appropriate location of a body, for example by surgery such as arthroscopy. The scaffold may then recruit endogenous stem cells and/or progenitor cells in vivo, i.e. from the host's body, in order to regenerate cartilage. Consequently, no injection of cells and/or no in vitro cells growth may be needed and the method of regeneration of cartilage may thus be cell-free.

Although the present invention has been described and illustrated in detail, it is clearly understood that the same is by way of illustration and example only and is not to be taken by way of limitations, the scope of the present invention being limited only by the terms of the appended claims.

## Claims

1. A composition to produce scaffold material adapted to regenerate cartilage, the composition including:
• Functionalized nanocrystalline cellulose including a plurality of first reactive moieties,
• Functionalized gelatine peptide chains including a plurality of second reactive moieties adapted to react with the first reactive moieties.

2. The composition according to the previous claim, wherein the first reactive moiety is a nucleophile, for example chosen among amine, a stabilized carbanion, a thiolactone and preferably a thiol.

3. The composition according to any of the previous claims, wherein the functionalized nanocrystalline cellulose includes a plurality of linkers bearing the first reactive moieties and chosen among an amino acid, a peptide, cysteamine, 3-amino-1,2,4-triazole-5-thiol and 6-amino-1-hexanethiol.

4. The composition according to the previous claim, wherein the linker is an amino acid and preferably a cysteine.

5. The composition according to any of the previous claims, wherein the functionalized nanocrystalline cellulose is under the form of crystals including faces and wherein the first reactive moieties are located on a single face of each of the crystals.

6. The composition according to any of the previous claims, wherein each of the second reactive moieties of the functionalized gelatine peptide chains comprises a double bond or a triple bond, for example a carbon-carbon double bond or carbon-carbon triple bond, preferably activated.

7. The composition according to any of the previous claims, wherein the second reactive moieties of the functionalized gelatine peptide chains are chosen among norbornene, norbornene-derivative, acrylate, methacrylate, α,β-unsaturated aldehyde or ketone, vinyl ester, vinyl sulfone, imidazole, and maleimide.

8. The composition according to any of the previous claims, wherein the weight percentage of functionalized gelatine peptide chains with regard to the composition is at most 10 %, preferably at most 5 %.

9. The composition according to any of the previous claims, wherein the weight percentage of functionalized cellulose with regard to the composition is 0.1 to 15 %, preferably 0.5 to 10 % and again preferably 1 to 5 %.

10. A scaffold material adapted to regenerate cartilages, the material including:
• Functionalized gelatine peptide chains
• Functionalized nanocrystalline cellulose
• A plurality of bridges cross-linking the functionalized nanocrystalline cellulose and the functionalized gelatine peptide chains.

11. A scaffold material according to the previous claim, wherein each bridge comprises at least one sulphur atom and/or at least one amino acid.

12. A scaffold material according to any of the previous claims, wherein the functionalized nanocrystalline cellulose is under the form of crystals including faces and wherein the bridges are connected to a single face of each of the crystals.

13. A method to form a scaffold adapted to regenerate cartilage, the method including:
• Providing a composition according to any of claims 1 to 9,
• Forming a scaffold shape
• Cross-linking the formed scaffold shape into a scaffold.

14. A method according to claim 13, wherein the step of forming a scaffold shape is performed by 3D-printing and/or the step of cross-linking is performed by a photo-click reaction.
